# EUROPÄISCHE PATENTSCHRIFT

(11) **EP 1 356 779 B1**
(45) Veröffentlichungstag und Bekanntmachung des Hinweises auf die Patenterteilung: **27.06.2007**
(21) Anmeldenummer: 03006683.1
(22) Anmeldetag: 26.03.2003
(51) Int. Cl.: A61B 18/14

(54) **Ablationsvorrichtung für Herzgewebe, insbesondere zur Erzeugung einer zirkulären Läsion um eine Gefässmündung im Herzen**
Cardiac tissue ablation device for the creation of a circular lesion in an infundibulum
Dispositif d'ablation cardiaque pour la création d'une lésion circulaire dans en infundibulum

(30) Priorität: 24.04.2002 DE 10218427
(43) Veröffentlichungstag der Anmeldung: 29.10.2003
(73) Patentinhaber: BIOTRONIK GmbH & Co. KG, 12359 Berlin (DE)
(72) Erfinder: Hintringer, Florian, Dr., 6060 Ampass (AT); Geistert, Wolfgang, Dr., 79618 Rheinfelden (DE)
(74) Vertreter: Lindner-Vogt, Karin L.

(56) Entgegenhaltungen:
- EP-A- 1 042 990
- WO-A-00/67656
- WO-A-01/37723
- WO-A-01/37925

## Beschreibung

Die Erfindung betrifft eine Ablationsvorrichtung für Herzgewebe, insbesondere zur Erzeugung einer zirkulären Läsion im Bereich einer Gefäßmündung im Herzen nach dem Oberbegriff des Patentanspruches 1.

Zum Hintergrund der Erfindung ist festzuhalten, dass die Katheterablation eine vermehrt eingesetzte Therapie zur Behandlung bestimmter Arrhythmien ist. Dabei wird an einer bestimmten Stelle im Herzmuskelgewebe mit Hilfe des Ablationsapplikators des Katheters eine Läsion - also eine Gewebedenaturierung in der Art einer Gewebevernarbung - erzeugt, um dort die für die Arrhytmien verantwortlichen, fehlerhaften elektrischen Reizleitungsbahnen zu unterbrechen. Die Energie-Einbringung in das Herzmuskelgewebe über den Ablationsapplikator erfolgt dabei in aller Regel über Ablationselektroden, die mit Hochfrequenzstrom arbeiten. Zur Ablation können ferner andere Energieformen, wie Mikrowellenenergie, Hochspannungsgleichstrom oder prinzipiell andere Denaturierungsmechanismen, wie Kälte oder Chemikalien (z.B. Alkohol), eingesetzt werden. Der Begriff "Ablationsapplikator", wie er in der vorliegenden Anmeldung auch in Verbindung mit dem eigentlichen Erfindungsgegenstand benutzt wird, soll grundsätzlich alle genannten Ablationsmöglichkeiten umfassen, wobei Ablationselektroden die gängigste Variante darstellen.

Aus einer Vielzahl von dem jeweiligen Anwendungszweck angepassten Varianten von Ablationskathetern sei als Stand der Technik die WO 98/49957 A1 herausgegriffen, die eine Ablationsvorrichtung zur Erzeugung linearer Läsionen zwischen den Mündungsöffnungen zweier Pulmonalvenen in den Vorhof des Herzens zeigt. Entsprechend dem Oberbegriff des Patentanspruches 1 ist dabei ein steuerbarer Katheter vorgesehen, der an seinem distalen Ende eine Widerlagervorrichtung in Form eines dilatierbaren Ballons zur Festlegung des distalen Endes im Ostium der Pulmonalvene trägt.

Bei dieser bekannten Ablationsvorrichtung dient der Katheter nicht nur zur Basispositionierung des Ablationsapplikators, sondern er trägt die entsprechenden Ablationselektroden selbst auf seinem Schaft. In seiner speziellen Ausgestaltung kann nun proximal von den Ablationselektroden der Schaft des Katheters durch eine zweite Führungseinrichtung vor die Mündungsöffnung einer zweiten Pulmonalvene verbracht werden, sodass sich die linear aneinander gereihten Ablationselektroden auf die Verbindungslinie zwischen den beiden Mündungsöffnungen zweier benachbarter Pulmonalvenen legen. Damit ist auf zuverlässige Weise eine lineare Läsion zwischen den beiden Mündungsöffnungen anzubringen.

Weitere Konfigurationen von Ablationskathetern sind beispielsweise der US 5,239,999 A, der WO 95/15115 A1 bzw. der WO 95/31111 A1 entnehmbar. Dort sind Ablationselektroden in unterschiedlich gewendelter oder leicht gebogener Form gezeigt. Der nächstkommende Stand der Technik ist durch die WO 00/67656 repräsentiert, aus der eine Ablationsvorrichtung gemäß dem Oberbegriff des Anspruches 1 bekannt ist.

Jüngere Untersuchungen haben gezeigt, dass gerade zur Therapie des arterialen Herzflimmerns zirkuläre Läsionen um die oder an den Mündungen der Pulmonalvenen (im Folgenden: PV-Mündung) in das Atrium hinein gute Erfolge erzielen.

Die letztgenannte Ablationsvorrichtung ist für derartige geformte Läsionen zwar praktisch geeignet, da eine ringförmige Anlage der Ablationselektroden um oder an der PV-Mündung realisierbar ist. Allerdings erscheint der Katheter in seinem mechanischen Aufbau noch verbesserbar. Insofern liegt der Erfindung die Aufgabe zugrunde, eine in ihrem Aufbau verbesserte Ablationsvorrichtung anzugeben, mit der auf zuverlässige und applikationstechnisch einfache Weise eine zirkuläre Läsion um eine oder in einer Gefäßmündung im Herzen erzeugt werden kann.

Diese Aufgabe wird durch die im Anspruch 1 angegebenen Merkmale gelöst. Demnach ist ein Katheter mit einem linearen Ablationsapplikator vorgesehen, der proximal der Widerlagervorrichtung des Katheters angeordnet und von einer gestreckten Passivstellung in eine radial expandierte, kreisbogenartig umlaufende Ablationsstellung verbringbar und vorzugsweise axial gegenüber der Widerlagervorrichtung verschiebbar ist.

Durch diese Ausgestaltung des Katheters wird einerseits eine zuverlässige Positionierung durch die Festlegung der Ablationsvorrichtung in der Gefäßmündung erreicht. Andererseits wird durch den in Ablationsstellung kreisbogenartig umlaufenden Ablationsapplikator dieser quasi forminhärent in eine korrekte Stellung zur Anbringung der zirkulären Läsion vorzugsweise bei Anordnung des Applikators proximal vor der Widerlagervorrichtung um die Gefäßmündung verbracht. Insoweit wird eine hohe Applikationssicherheit unter entsprechender Verbesserung des Therapieerfolges erzielt.

Die Erfindung sieht dabei eine Kombination zweier Katheter vor, nämlich eines steuerbaren Positionierkatheters, der an seinem distalen Ende mit der Widerlagervorrichtung versehen ist, und eines damit gekoppelten Ablationskatheters, der an seinem distalen Ende den Ablationsapplikator trägt. Durch diese funktionale Trennung kann der Positionierkatheter sehr kompakt und damit flexibel und gut steuerbar ausgestaltet werden, was insbesondere der Positionierbarkeit in Gefäßöffnungen zugute kommt.

Für die Koppelung von Positionier- und Ablationskatheter ist dabei erfindungsgemäß eine Frührung des Ablationskatheters vorgesehen, die nach Art eine Mono-Rail-Führung eines Katheters auf einem Führungsdraht ausgebildet ist. Dazu ist der Ablationskatheter in seinem distalen Endbereich mittels zweier Führungshülsen verschiebbar auf dem Positionierkatheter geführt. Die Hülsen sind beiderseits des Ablationsapplikators angeordnet, wobei durch axiales Verschieben der Hülsen aufeinanderzu der Ablationsapplikator in seine Ablationsstellung verbringbar ist. Dies wird vorzugsweise durch eine Zugdrahtkinematik vorgenommen.

Bei der Widerlagervorrichtung handelt es sich vorzugsweise um einen dilatierbaren Ballon am Schaft des Positionierkatheters. Der Ballondurchmesser in inflatiertem Zustand ist dabei an den Durchmesser des ihn aufnehmenden Gefäßes anzupassen, d. h. er liegt in einer Größenordnung von ca. 5 bis 25 mm.

Um die Bildung der kreisbogenartig umlaufenden Ablationsstellung zu unterstützen, ist der Ablationsapplikator vorzugsweise durch eine Mehrfach-Elektrodenanordnung gebildet, deren in Axialrichtung aneinandergereihte Einzelelektroden aus einem hochflexiblen Werkstoff- beispielsweise jeweils aus einer Spiralwicklung oder aus flexiblem, leitenden Kunststoff - bestehen.

Durch eine Überdeckung von mindestens 180° durch den Ablationsapplikator wird gewährleistet, dass eine komplett umlaufende zirkuläre Läsion mit nur einer Verdrehung des Ablationskatheters erzielbar ist.

Wenngleich dies nicht umittelbar Gegenstand der Erfindung ist, ist darauf hinzuweisen, dass insbesondere der steuerbare Positionierkatheter mit bekannten Maßnahmen zur Kontrolle der korrekten Lage versehen sein kann. So kann die Lage sonographisch über einen an der Spitze des Positionierkatheters angeordneten Ultraschallwandler oder über ein bipolares Elektrogramm kontrolliert werden, das durch eine bipolare Elektrodenanordnung an der Spitze des Positionierkatheters abgeleitet werden kann. Auch kann der Positionierkatheter über zusätzliche Lumen zur Injektion von Röntgenkontrastmittel verfügen, das über das Lumen in die Pulmonalvene zu deren angiographischer Darstellung injiziert wird.

Weitere Merkmale, Einzelheiten und Vorteile der Erfindung ergeben sich aus der nachfolgenden Beschreibung, in der ein Ausführungsbeispiel des Erfindungsgegenstandes anhand der beigefügten Zeichnungen näher erläutert werden. Es zeigen:
- Fig. 1: eine schematische Teildarstellung einer Ablationsvorrichtung in Passivstellung in einer ersten Ausführungsform, und
- Fig. 2: eine Darstellung analog Fig. 1 in Ablationsstellung der Vorrichtung.

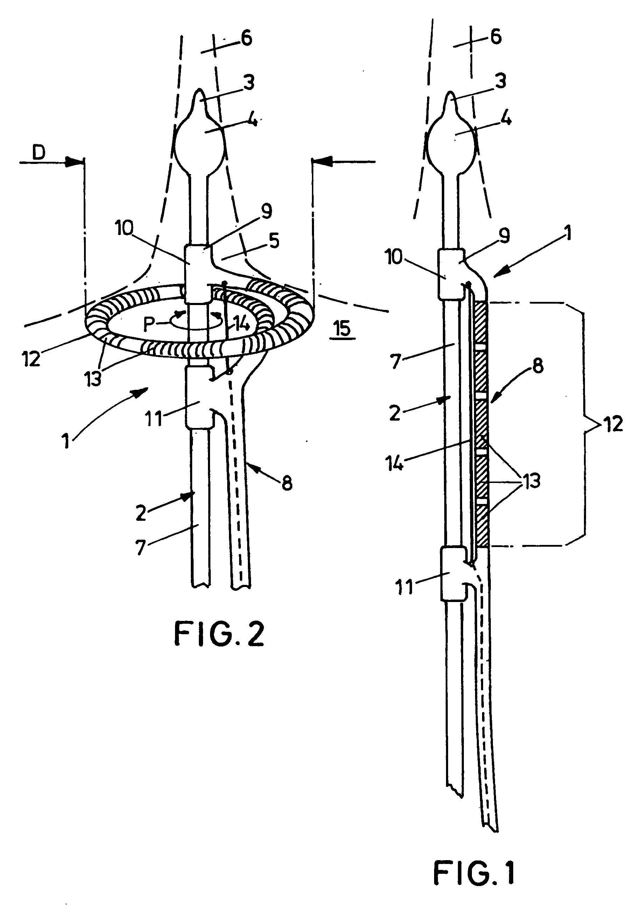

Wie aus Fig. 1 deutlich wird, weist die als Ganzes mit 1 bezeichnete Ablationsvorrichtung einen steuerbaren Positionierkatheter 2 auf, der an seinem distalen Ende 3 mit einem dilatierbaren Ballon 4 versehen ist. In allen Fig. 1 bis 3 ist der Ballon 4 in seinem aufgeweiteten Zustand gezeigt, in dem er das distale Ende 3 des Katheters 2 in einer in Fig. 1 und 2 strichliert dargestellten Mündungsöffnung 5 einer Pulmonalvene 6 in das Atrium des Herzens festlegt. Der Positionierkatheter 2 kann üblicher Bauart sein und beispielsweise mit einem Lumen für einen Führungsdraht, einer Deflektionseinrichtung zur gezielten Steuerung des distalen Endes 3 usw. versehen sein. Weitere Zusatzausrüstungen wurden ferner in der Beschreibungseinleitung bereits genannt.

Auf dem Schaft 7 des Positionierkatheters 2 ist ein Ablationskatheter 8 angeordnet. Zur verschiebbaren Führung des Ablationskatheters 8 auf dem Positionierkatheter 2 sind dabei zwei an den Ablationskatheter 8 am distalen Ende 9 bzw. einige Zentimeter davor in proximaler Richtung entfernt angeformte Führungshülsen 10, 11 vorgesehen, die auf dem Positionierkatheter 2 gleitend verschoben werden können.

Im Bereich zwischen den beiden Führungshülsen 10, 11 ist am Ablationskatheter 8 ein Ablationsapplikator 12 in Form von fünf aneinandergereihten Ringelektroden 13 vorgesehen, die jeweils aus hochflexiblem Spiraldraht bestehen. Über diese Ringelektroden 13 kann ein Hochfrequenzstrom an damit in Kontakt gelangendes Gewebe abgegeben werden, um eine Läsion zu erzeugen.

In Fig. 1 ist die gestreckte Lage des Ablationsapplikators 12 dargestellt, aus der er mit Hilfe eines im Ablationskatheter 8 verlaufenden Zugdrahtes 14 in die in Fig. 2 gezeigte, radial expandierte Ablationsstellung verbringbar ist. Dazu ist der Zugdraht 14 im Bereich der distalen Führungshülse 10 befestigt und läuft im Bereich der proximalen Führungshülse 11 in den Ablationskatheter 8 ein. Durch Gegenhalten am Zugdraht 14 und durch Vorschieben des Ablationskatheters 8 wird die proximale Führungshülse 11 am Positionierkatheter 2 entlang in distaler Richtung vorgeschoben, sodass sich der Ablationskatheter 8 im Bereich des Ablationsapplikators 12 aufweitet und in eine kreisbogenartig umlaufende Konfiguration durch entsprechende Vorformung des Ablationskatheters 8 verbracht wird. Der Ablationsapplikator 12 überdeckt dabei einen Peripherwinkel P von mehr als 180°, sodass sich in dieser Ablationsstellung der Ablationsapplikator 12 über mehr als die Hälfte des Umfangs der zu erzeugenden zirkulären Läsion erstreckt.

Im Folgenden soll kurz die Erzeugung der zirkulären Läsion anhand der Fig. 1 und 2 dargestellt werden. So wird der Positionierkatheter 2 bei nicht dilatiertem Ballon 4 über eine transseptale Punktion in den linken Vorhof des Herzens eingebracht, wo mit üblichen Mitteln die Einmündungen aller Pulmonalvenen sondiert werden. Nach Bestätigung der korrekten Lage des distalen Endes 3 des Positionierkatheters 2 in der Mündungsöffnung 5 der gewünschten Pulmonalvene 6 wird der Ballon 4 dilatiert und damit der Positionierkatheter 2 in der Mündungsöffnung 5 festgelegt.

Anschließend wird der Ablationskatheter 8 über den Schaft 7 des Positionierkatheters 2 vorgeschoben, bis die in Fig. 1 dargestellte Position vor dem distalen Ende 3 des Positionierkatheters 2 erreicht ist. Der Ablationsapplikator 12 liegt also proximal vom Ballon 4 im Bereich des Vorhofes 15 vor der Mündungsöffnung 5. Durch relatives Verschieben von Ablationskatheter 8 und Zugdraht 14 wird die proximale Führungshülse 11 vorgeschoben und der Ablationsapplikator 12 in die Ablationsstellung gemäß Fig. 2 gebracht, in der die Ringelektroden 13 über den Peripherwinkel P am Endocard im Vorhof anliegen. Durch Abgabe eines hochfrequenten Stromes wird ein Teil der zirkulären Läsion erzielt. Anschließend wird der Ablationsapplikator 12 gegebenenfalls teilweise in die in Fig. 1 gezeigte Passivstellung verbracht, um ca. 180° gedreht und wieder kreisbogenartig in die in Fig. 2 gezeigte Ablationsstellung aufgeweitet. Auch ein Zurückziehen, Drehen und wieder Vorschieben des Applikators 12 in gebogenem Zustand ist möglich. Damit liegt der Ablationsapplikator 12 in dem Bereich am Endocard des Vorhofes an, der vorher noch nicht mit einer Läsion versehen worden ist. Durch eine nochmalige Stromabgabe wird die zirkuäre Läsion um die Mündungsöffnung 5 herum geschlossen.

Da die Normalgröße des linken Vorhofes eines Herzens ca. 40 mm beträgt, aber bei Patienten mit Vorhofflimmern auch deutlich höhere Werte, wie 60 und mehr mm erreicht werden, sollte der wirksame Durchmesser des Ablationsapplikators 12 in Ablationsstellung 5 bis 25 mm für kleine Blutgefäße (z. B. die untere Lungenvene) betragen. Für vergrößerte Atrien- bzw. Pulmonalvenen sind wirksame Durchmesser von 25 bis 60 mm anzusetzen.

## Patentansprüche

1. Ablationsvorrichtung für Herzgewebe, insbesondere zur Erzeugung einer zirkulären Läsion um eine Gefäßmündung (5) im Herzen, umfassend
- einen Katheter (2), der im Bereich seines distalen Endes (3) mit einer Widerlagervorrichtung (4) zum Halten des distalen Endes (3) an einer kardialen Gefäßmündung (5) versehen ist,
- einen linearen Ablationsapplikator (12), der proximal vor der Widerlagervorrichtung (4) des Katheters (2) angeordnet und von einer gestreckten Passivstellung in eine radial expandierte, etwa kreisbogenartig umlaufende Ablationsstellung verbringbar ist,
- einen steuerbaren Positionierkatheter (2), der an seinem distalen Ende (3) mit der Widerlagervorrichtung (4) versehen ist, und
- einen mit dem Positionierkatheter (2) gekoppelten Ablationskatheter (8), der mit dem Ablationsapplikator (12) an seinem distalen Ende (9) versehen ist, der in seiner Ablationsstellung den Positionierkatheter (2) etwa kreisbogenartig umgibt,
**dadurch gekennzeichnet, dass** der Ablationskatheter (8) zumindest in seinem distalen Endbereich mittels zweier Führungshülsen (10, 11) verschiebbar auf dem Positionierkatheter (2) geführt ist, die beiderseits des Ablationsapplikators (12) angeordnet sind und durch axiales Verschieben aufeinander zu den Ablationsapplikator (12) in die Ablationsstellung verbringen.

2. Ablationsvorrichtung nach Anspruch 1, **dadurch gekennzeichnet, dass** der Ablationsapplikator (12) axial gegenüber der Widerlagervorrichtung (4) verschiebbar ist.

3. Ablationsvorrichtung nach Anspruch 1 oder 2, **dadurch gekennzeichnet, dass** die Widerlagervorrichtung durch einen dilatierbaren Ballon (4) am Schaft (7) des Katheters (2) gebildet ist.

4. Ablationsvorrichtung nach einem der vorgenannten Ansprüche, **dadurch gekennzeichnet, dass** die Steuerung des Ablationsapplikators (12) von der Passiv- in die Ablationsstellung durch eine Zugdrahtkinematik (14) vornehmbar ist.

5. Ablationsvorrichtung nach einem der vorgenannten Ansprüche, **dadurch gekennzeichnet, dass** der Ablationsapplikator (12) durch eine Mehrfach-Elektrodenanordnung gebildet ist, deren in Axialrichtung aneinandergereihte Einzelelektroden (13) aus einem hochflexiblen Werkstoff bestehen.

6. Ablationsvorrichtung nach einem der vorgenannten Ansprüche, **dadurch gekennzeichnet, dass** der Ablationsapplikator (12) in seiner kreisbogenartig umlaufenden Ablationsstellung einen Peripherwinkel (P) von mindestens 180° überdeckt.

## Claims

1. An ablation device for cardiac tissues, in particular for producing a circular lesion around a vascular orifice (5) in the heart, comprising
- a catheter (2) which is provided in the region of its distal end (3) with a stop device (4) for retaining the distal end (3) on a cardiac vascular orifice (5),
- a linear ablation applicator (12) which is arranged proximally in front of the stop device (4) of the catheter (2), and can be displaced from an extended positive position to a radially expanded ablation position running approximately in the shape of the arc of a circle,
- a controllable positioning catheter (2) which is provided on its distal end (3) with the stop device (4), and
- an ablation catheter (8) coupled to the positioning catheter (2), which catheter is provided at its distal end (9) with the ablation applicator (12) which in its ablation position surrounds the positioning catheter (2) approximately in the shape of the arc of a circle, **characterised in that** the ablation catheter (8) can be guided displaceably on the positioning catheter (2), at least in its distal end region, by means of two guide sleeves (10, 11), which sleeves are arranged on both sides of the ablation applicator (12) and move to the ablation position due to axial displacement, one on top of the other, the ablation applicator (12), to the ablation position.

2. The ablation device according to Claim 1, **characterised in that** the ablation applicator (12) is axially displaceable relative to the stop device (4).

3. The ablation device according to Claim 1 or 2, **characterised in that** the stop device is formed by a dilatable balloon (4) on the shaft (7) of the catheter (2).

4. The ablation device according to one of the preceding claims, **characterised in that** the ablation applicator (12) can be controlled from the passive to the ablation position by tension wire kinematics (14).

5. The ablation device according to one of the preceding claims, **characterised in that** the ablation applicator (12) is formed by a multiple electrode arrangement whose individual electrodes (13), arranged in the axial direction, consist of a highly flexible material.

6. The ablation device according to one of the preceding claims, **characterised in that** the application applicator (12) overlaps a peripheral angle (P) of at least 180° in its ablation position running approximately in the shape of the arc of a circle.

## Revendications

1. Dispositif d'ablation pour tissu cardiaque, notamment pour la génération d'une lésion circulaire autour d'une embouchure de vaisseau (5) du coeur, comprenant
- un cathéter (2) qui est pourvu au niveau de son extrémité distale (3) d'un dispositif de contre-palier (4) pour maintenir l'extrémité distale (3) sur une embouchure de vaisseau cardiaque (5),
- un applicateur d'ablation linéaire (12) qui est disposé au niveau proximal avant le dispositif de contre-palier (4) du cathéter (2) et peut être amené d'une position passive étirée à une position d'ablation en expansion radiale périphérique approximativement en arc de cercle,
- un cathéter de positionnement contrôlable (2) qui est pourvu à son extrémité distale (3) du dispositif de contre-palier (4) et
- un cathéter d'ablation (8) couplé au cathéter de positionnement (2) qui est pourvu à son extrémité distale (9) de l'applicateur d'ablation (12) et qui, dans sa position d'ablation, entoure approximativement en arc de cercle le cathéter de positionnement (2),
**caractérisé en ce que** le cathéter d'ablation (8) est guidé du moins dans sa zone terminale distale de manière mobile sur le cathéter de positionnement (2) au moyen de deux manchons de guidage (10, 11) qui sont disposés des deux côtés du cathéter d'application (12) et, par décalage axial l'un vers l'autre, amènent le cathéter d'ablation (8) en position d'ablation.

2. Dispositif d'ablation selon la revendication 1, **caractérisé en ce que** l'applicateur d'ablation (12) est mobile axialement par rapport au dispositif de contre-palier (4).

3. Dispositif d'ablation selon la revendication 1 ou 2, **caractérisé en ce que** le dispositif de contre-palier (4) est constitué par un ballon dilatable (4) sur la tige (7) du cathéter (2).

4. Dispositif d'ablation selon une des revendications précédentes, **caractérisé en ce que** la commande de l'applicateur d'ablation (12) le faisant passer de la position passive à la position d'ablation peut être assurée par une cinématique à fil de traction (14).

5. Dispositif d'ablation selon une des revendications précédentes, **caractérisé en ce que** l'applicateur d'ablation (12) est constitué par un dispositif d'électrodes multiples dont les électrodes distinctes (13) alignées entre elles dans le sens axial sont composées d'une matière très souple.

6. Dispositif d'ablation selon une des revendications précédentes, **caractérisé en ce que** l'applicateur d'ablation (12), dans sa position d'ablation périphérique en forme d'arc de cercle, couvre un angle périphérique (P) d'au moins 180°.
